# EUROPÄISCHE PATENTSCHRIFT

(11) **EP 1 240 904 B1**
(45) Veröffentlichungstag und Bekanntmachung des Hinweises auf die Patenterteilung: **28.04.2010**
(21) Anmeldenummer: 02003625.7
(22) Anmeldetag: 16.02.2002
(51) Int. Cl.: A61K 38/57, A61P 37/06

(54) **Verwendung eines C1-Esterase-Inhibitors zur Verhinderung oder Verzögerung der Abstossung von Xenotransplantaten in Säugetieren**
Use of a C1-esterase inhibitor for preventing or delaying xenotransplant rejection in mammals
Utilisation d'un inhibiteur de C1-estérase pour la prévention ou le délai de rejets de xénogreffes chez les mammifères

(30) Priorität: 14.03.2001 DE 10112617
(43) Veröffentlichungstag der Anmeldung: 18.09.2002
(73) Patentinhaber: CSL Behring GmbH, 35041 Marburg (DE)
(72) Erfinder: Winkler, Michael, 30996 Hemmingen (DE); Rueckoldt, Horst, 31275 Lehrte (DE)

(56) Entgegenhaltungen:
- WO-A-01/57079
- MATSUNAMI K ET AL: "A surface-bound form of human C1 esterase inhibitor on xenografts: the complement regulatory function." TRANSPLANTATION PROCEEDINGS, (2000 AUG) 32 (5) 901-2., XP002208304
- MARX G ET AL: "Septic shock after liver transplantation for Caroli's disease: clinical improvement after treatment with C1- esterase inhibitor." INTENSIVE CARE MEDICINE. REF: 10, Bd. 25, Nr. 9, September 1999 (1999-09), Seiten 1017-1020, XP002208305
- DATABASE CA [Online] CHEMICAL ABSTRACTS SERVICE, COLUMBUS, OHIO, US; MIYAKAWA, SHUJI: "A membrane-bound form of human and mouse complement C1 esterase inhibitor for improved xenograft rejection suppression" Database accession no. 134:146402 XP002208309 & JP 2001 029074 A (OSAKA UNIVERSITY, JAPAN) 6. Februar 2001 (2001-02-06)
- MATSUNAMI K ET AL: "A surface-bound form of human C1 esterase inhibitor improves xenograft rejection." TRANSPLANTATION, (2000 MAR 15) 69 (5) 749-55., XP001089617
- SCHELZIG H ET AL: "Ex-vivo hemoperfusion (eHPS) of pig-lungs with whole human blood: effects of complement inhibition with a soluble C1- esterase - inhibitor." ANNALS OF TRANSPLANTATION, (2001) 6 (3) 34-9., XP001089618
- SCHELZIG, H. (1) ET AL: "Pig to human lung transplantation: Induction therapy with soluble C1- esterase - inhibitor (C1-INH." XENOTRANSPLANTATION, (AUGUST, 2001) VOL. 8, NO. SUPPLEMENT 1, PP. 66. PRINT. MEETING INFO.: VI CONGRESS OF THE INTERNATIONAL XENOTRANSPLANTATION ASSOCIATION CHICAGO, ILLINOIS, USA SEPTEMBER 29-OCTOBER 03, 2001, XP001086434
- VANGEROW B ET AL: "C1-Inhibitor for treatment of acute vascular xenograft rejection in cynomolgus recipients of h-DAF transgenic porcine kidneys." XENOTRANSPLANTATION, Bd. 8, Nr. 4, November 2001 (2001-11), Seiten 266-272, XP002208306
- FIANE ARNT E ET AL: "C1-inhibitor attenuates hyperacute rejection and inhibits complement, leukocyte and platelet activation in an ex vivo pig-to-human perfusion model." IMMUNOPHARMACOLOGY, Bd. 42, Nr. 1-3, Mai 1999 (1999-05), Seiten 231-243, XP002208307 ISSN: 0162-3109
- STRUBER M ET AL: "C1-esterase inhibitor in graft failure after lung transplantation." INTENSIVE CARE MEDICINE, Bd. 25, Nr. 11, November 1999 (1999-11), Seiten 1315-1318, XP002208308

## Beschreibung

Gegenstand der Erfindung ist die Verwendung einer einen C1-Esterase-Inhibitor (C1-INH) enthaltenden Zubereitung zur Verbesserung der Erfolgsaussichten bei der Xenotransplantation.

Die Xenotransplantation könnte wegen des Mangels an geeigneten Spenderorganen in Zukunft erhebliche Bedeutung in der medizinischen Operationstechnik erlangen, wenn es gelingt, die natürlichen Abstoßungs- und Abwehrreaktionen bei einer diskordanten Xenotransplantation zu überwinden. Derzeit stehen allerdings die hyperakute Abstoßung von Xenotransplantaten (HAR) und die akute Abstoßung von Xenotransplantaten (AVR) einem dauerhaften Erfolg einer Transplantation von Organen unterschiedlicher Arten von Säugetieren noch entgegen. Weder die durch vorgebildete Antikörper verursachte sofortige Abstoßung (HAR) noch die durch erst nach der Transplantation gebildete Antikörper verzögert auftretende Abstoßung (AVR) kann derzeit bei der Xenotransplantation überwunden werden. Auch immunsuppresive Maßnahmen wie die Verabreichung von Cyclosporin A, von Mycophenolat-Mofetil oder von Steroiden können eine Aktivierung des Komplementsystems und eine dadurch entstehende Zellschädigung nicht verhindern. Es stellte sich deshalb die Aufgabe, durch eine dauerhafte Desaktivierung des Komplementsystems einen Weg zu finden, der eine anhaltende Verträglichkeit des Xenotransplantats mit dem Empfängerorganismus ermöglicht.

Der C1-Inhibitor, auch als C1-Esterase-Inhibitor bezeichnet, ist ein im Blut vorhandenes Protein und ist der Hauptinhibitor des klassischen Weges des Komplementsystems und des Kontaktsystems. Der C1-Inhibitor kann die aktivierte Form des Faktors XII und des Kallikreins hemmen [1, 2, 3, 4, 5, 6, 7, 8]. Der C1-Inhibitor reguliert somit die Aktivitäten von zwei Plasmakaskaden, nämlich das Komplementsystem und das Kontaktsystem, durch die biologisch aktive Peptide erzeugt werden. Der C1-Inhibitor ist deshalb auch ein wichtiger Regulator des Entzündungssystems. Außerdem hemmt der C1-Inhibitor den aktivierten Faktor XI [9, 10]. Hieraus ergibt sich, dass der C1-Inhibitor als ein Coagulations-Hemmstoff betrachtet werden kann. Auch der Gewebeplasminogen-Aktivator und Plasmin werden in gewissem Ausmaß von dem C1-Inhibitor gehemmt, obwohl das nicht seine Hauptfunktion ist [11, 12].

Der C1-Inhibitor wird in erheblichem Ausmaß durch Reinigung aus Plasma gewonnen und für klinische Anwendungen benutzt, insbesondere bei der Behandlung des hereditären Angioödems, einer Erkrankung, die durch einen genetisch bedingten Mangel des C1-Inhibitors hervorgerufen wird. Außerdem ist bereits beschrieben worden, dass durch Verabreichung des C1-Inhibitors bei systemischen Entzündungen [42], bei schweren Verbrennungen, Pankreatitis, Knochenmarkstransplantationen, der Zytokin-Therapie und beim Einsatz in extrakorporalen Blutkreisläufen [39] gute therapeutische Erfolge erzielt wurden.

Die vollständige genomische und die cDNA, die für den C1-Inhibitor kodiert, ist bereits kloniert worden [13, 14]. Verschiedene Varianten des rekombinanten C1-Inhibitors mit Aminosäuremutationen in der P1 und den P3 und/oder P5-Positionen des reaktiven Zentrums als auch Varianten, die aus Patienten mit einem hereditären Angioödem isoliert wurden, sind bereits rekombinant hergestellt worden [15, 16, 17, 40, 18, 19, 20].

Der C1-Inhibitor gehört zu der großen Familie der Serin-Proteinase Inhibitoren, die auch Serpine genannt werden [21, 22]. Auf SDS-Polyacrylamidgelen zeigt der C1-Inhibitor ein Molekulargewicht von etwa 105 KD. Seine Plasmakonzentration liegt bei etwa 270 mg/l [23, 24]. Der C1-Inhibitor ist ein Protein, dessen Plasmaspiegel bei unkomplizierten Infektionen und anderen Entzündungen bis zum zweifachen ansteigen kann [25]. Die vermehrte Bildung des C1-Inhibitors bei Entzündungen dient wahrscheinlich dem Schutz des Organismus gegen die schädlichen Wirkungen der intravaskulären Aktivierung des Komplementsystems und des Kontaktsystems während der akuten Reaktionen.

Die Serpine reagieren als Inhibitoren durch Bildung bimolekularer Komplexe mit der zu hemmenden Proteinase. Bei diesen Komplexen wird das aktive Zentrum der Proteinase durch das aktive Zentrum des Serpins gebunden und damit inaktiv [26]. Die Serpine reagieren spezifisch mit bestimmten Proteinasen, wobei diese Spezifität durch die Aminosäuresequenzen des reaktiven Zentrums bestimmt wird.

Die vorliegende Erfindung geht nun von der Beobachtung aus, dass die Abstoßung eines Xenotransplantats durch die Verabreichung eines C1-Inhibitors blockiert werden kann.

Gegenstand der Erfindung ist deshalb die Verwendung einer einen humanen C1-Esterase-Inhibitor (C1-INH) enthaltenden Zubereitung zur Verhinderung oder Verzögerung der hyperakuten und/oder akuten Abstoßung von Xenotransplantaten in Säugetieren, bevorzugterweise bei Menschen.

Dabei wird bei der Transplantation eines Organs eines Säugetieres auf einen Primaten ein humaner C1-Esterase-Inhibitor verwendet. Dabei wird so vorgegangen, dass dem empfangenden Organismus bereits vor der Transplantation eine therapeutisch wirksame Menge der C1-INH-Zubereitung verabreicht wird und er nach erfolgter Transplantation tägliche Dosierungen der C1-INH-Zubereitung in einer Menge erhält, die zur Verhinderung einer Abstoßung des Transplantats ausreicht. Vor der Transplantation wird im allgemeinen eine Menge an C1-INH von nicht mehr als 150 I.U./kg Körpergewicht verabreicht. Nach erfolgter Transplantation beträgt die verabreichte Menge an C1-INH im allgemeinen nicht mehr als 80 I.U./kg Körpergewicht.

Als Modell für erfindungsgemäße Transplantationsverfahren wurde die Übertragung eines Organs eines Schweins auf einen Primaten untersucht. Der Fachmann kann mit den gefundenen Erkenntnissen auch die Xenotransplantation beim Menschen ermöglichen.

Es hat bereits einige Versuche gegeben, die Abstoßung von Xenotransplantaten zu verhindern. So ist in der internationalen Patentanmeldung WO 97/16064 [41] ein Verfahren zur Xenotransplantation beschrieben, bei dem transgene Gewebe oder Organe in den menschlichen Empfängerorganismus eingeführt werden, wodurch eine Verminderung der hyperakuten Abstoßungsreaktion (HAR) im Vergleich zu menschlichen Empfängerorganismen, die nicht-transgene Gewebe oder Organe erhalten, beobachtet wird. Es gibt auch bereits Perfusionsstudien, in denen gezeigt wurde, dass die Überlebenszeit von Schweinenieren, die mit frischem menschlichen Blut perfundiert wurden, das den C1-INH enthielt, verlängert werden konnte [38]. Es wurde vermutet, dass die Milderung der hyperakuten Abstoßung (HAR) wahrscheinlich durch die Inhibition des Komplements bedingt war. Überraschenderweise lässt sich dieses Verfahren nun auch auf transplantierte Organe übertragen, wenn nach entsprechender Vorbehandlung im Empfängerorga-nismus kontinuierlich für eine ausreichend hohe Dosis C1-INH gesorgt wird.

Folgende Untersuchungen wurden durchgeführt:

### Materialien und Verfahren

### Tiere:5 Cynomolgus-Affen (macaca fascicularis)

mit einem Gewicht von 3,3 bis 4,7 kg und einem Alter von 4,5 bis 5 Jahren wurden teils für pharmakokinetische Untersuchungen (n = 2) oder für Nieren-Xenotransplantationen (n = 3) verwendet. Die Affen waren vom Deutschen Primatenzentrum in Göttingen gekauft worden und mit negativem Befund auf Macaque Herpes B (Cercopithecine herpes virus type 1), Ebola, Marburg, Simian T-cell Leukemia Virus (STLV), Simian Retrovirus D (SRV) und Simian Immunodeficiency Virus (SIV) getestet worden.

Für die Xenotransplantationsversuche wurden 3 nicht-vorbehandelte große weiße Schweine (Schweinezuchtverband Weser-Ems, Oldenburg, Deutschland) als Spendertiere verwendet. Die Schweine waren 8 bis 18 Wochen alt und wogen 18 bis 23 kg. Alle chirurgischen Maßnahmen und die nachoperative Behandlung wurden in Übereinstimmung mit dem nationalen Deutschen Institut für Tierpflege durchgeführt und waren von den örtlichen Behörden für Tierfürsorge gebilligt.

### Nieren-Xenotransplantation

Die Entnahme des Spenderorgans und die Transplantation der Xeno-Niere unter Erhalt der funktionsunfähigen eigenen Nieren des Empfängers und ihre Verbindung mit seiner Harnblase wurde nach bekannten Verfahren durchgeführt. Der Verschluss der natürlichen Harnleiter mit bei der Operation angebrachten Ligaturen wurde sowohl durch Ultraschall oder NMR als auch durch Autopsie geprüft. Die Milz wurde nicht entfernt. Zur postoperativen, intravenösen Arzneimitteltherapie wurde bei allen Affen in die rechte innere Halsvene ein Anschlusssystem implantiert.

### Definition der HAR und AVR

Unter der HAR des Fremdtransplantats wurde die komplette Thrombose des Transplantats mit Gefäßverbindungen verstanden, die 2 Stunden nach Wiederdurchströmung bei Durchführung einer Biopsie keine Blutungen zeigten. Zusätzlich mussten sowohl bei einer histologischen Untersuchung als auch bei immuno-histochemischen Untersuchungen Anzeichen von HAR [27, 28, 29] in den Standard-HE-Abschnitten vorhanden sein. Die AVR wurde aufgrund von klinischen und histologischen Parametern diagnostiziert. Nachdem alle nicht auf die Gewebsabstoßung zurückzuführenden Gründe für eine Verschlechterung der Transplantatfunktionen wie Sepsis oder technische Fehler (durch Ultraschall) ausgeschlossen worden waren, wurde jedes Ereignis, das mit einem Anwachsen des Kreatininspiegels um 20% über den Grundwert verbunden war, der AVR zugeschrieben.

### Immunsuppressive Therapie

Die Immunsuppression bestand aus Cyclosporin, Mycophenolat-Mofetil und Prednisolon [30, 31, 32]. Cyclosporin wurde zunächst oral einen Tag vor der Transplantation in einer Dosierung von 100 mg/kg gegeben. Nach der Transplantation wurde CyA oral in einer Dosis verabreicht, die ausreichte, um einen Blutspiegel von 400 bis 600 ng/l zu erreichen, bei dem ausreichende Wirksamkeit und minimale Toxizität in dieser Primatenart erreicht wird. Die Verabreichung von MMF begann 3 Tage vor der Transplantation mit einer Magensonde. Die MMF-Dosierung wurde so eingestellt, dass ein MPA-Plasmaspiegel von 1 bis 4 µg/ml aufrecht erhalten wurde. Die Steroide wurden wie folgt verabreicht: 1 mg/kg Methylprednisolon wurde intravenös am Tag der Transplantation gegeben, gefolgt von einer peroralen Applikation von 1 mg/kg Prednisolon in der 1. Woche nach der Transplantation. Dann wurde jeden Tag um 0,05 mg/kg reduziert, um eine Erhaltensdosis von 0,2 mg/kg zu erreichen. Den Tieren Nr. 3 und Nr. 4 wurde auch Cyclophosphamid zur Induktionstherapie an den Tagen -1, 0, 2 und 4 in einer Menge von 40 mg/kg, 10 mg/kg bzw. 20 mg/kg i.V. verabreicht. Beim Tier Nr. 5 wurde keine Cyclophosphamid-Induktionstherapie durchgeführt.

### Postoperative Blutprüfung

Blutproben wurden jeden Tag zur Bestimmung der Vollblutzählung, des Harnstoffs, des Kreatinins und der Elektrolyte entnommen. Zusätzlich wurde in ausgewählten Tieren der 24 Stunden Urin gesammelt und bezüglich der Elektrolytkonzentrationen analysiert. Die Cyclosporinspiegel im Gesamtblut ebenso wie die MPA-Plasmaspiegel wurden mit Messmethoden unter Verwendung von monoklonalen Antikörpern bestimmt (EMIT 2000, Aventis Behring GmbH).

### Duchfluss-Cytometrie

Präoperative und postoperative Sera wurden auf ihren antiporcinen Antibodytiter mit einem Durchfluss-Cytometrie-Assay zur Analyse getestet. Nach Blutentnahme wurde das Serum durch Zentrifugation bei 3.400 x g für 10 Minuten bei 20°C hergestellt. Zum Nachweis von antiporcinen Antikörpern wurden gefrorene Aliquots von porcinen peripheren Blutleukozyten (PPBL), die aus einem individuellen großen weißen Schwein erhalten worden waren, aufgetaut und 0,5 x 10⁵ Zellen wurden durch 20 µl des entsprechenden Cynomolgus-Serums in verschiedenen Lösungen gefärbt. Nach 20 minütiger Inkubation bei 4°C wurden die Zellen zweimal mit PBS gewaschen, welches 1% BSA und 0,1% Natriumazid enthielt. Gebundene Cynomolgus-Antikörper wurden mit Ziegen-antihumanen FITC sekundären Antikörpern, die mit IgG (Dianova, Hamburg) oder IgM (Dianova, Hamburg) reagierten, nachgewiesen. Diese Antikörper sind dafür bekannt, dass sie mit Cynomolgus-Immunglobulinen kreuzreagieren [33]. Die Antikörper waren mit Schweineserum präabsorbiert worden. Nach einer Inkubation von 20 Minuten bei 4°C wurden die Zellen wieder zweimal gewaschen und dann auf einem FACScan (Becton Dickinson, Mountain View, CA) Cytometer analysiert.

### Analyse der Komplementspiegel

Zum Nachweis der Plasmakomplementspiegel wurde venöses Blut entnommen und EDTA als Antikoagulanz zugesetzt. Sofort nach der Blutentnahme wurden die Proben zentrifugiert; das Plasma wurde dann bei -70°C bis zur Analyse aufbewahrt. Die Konzentrationen von C3a und sC5b-9 im Cynomolgusplasma wurden mit handelsüblichen ELISA-Reagenzien (Quidel, San Diego, CA, USA), die für ihre Kreuzreaktion mit Cynomolguskomplementverbindungen bekannt sind, gemessen. Diese Untersuchungen wurden genau nach den Anweisungen der Hersteller durchgeführt.

### Analyse der C1-INH Aktivität

Sowohl die Gesamtaktivität von C1-INH als auch die Gesamtmenge des C1-INH-Proteins in Citratplasma wurde quantitativ gemessen. Die Aktivität von C1-INH wurde wie folgt gemessen: C1-INH in der Probe inhibiert ein definiertes Volumen von exogener C1-Esterase. Die übrigbleibende Aktivität der C1-Esterase wird durch Messung des Anstieges der Absorption bei 405 nm in einem kinetischen Test gemessen (Berichrom® C1-Inhibitor, Dade Behring Inc., Newark, USA). Die C1-Aktivität der Proben wurde aus einer Referenzkurve, die aus menschlichem Standardplasma hergestellt worden war, berechnet. Normale Werte (für Menschen) wurden nach einem Standard definiert, der von dem Hersteller des C1-INH-Assays geliefert worden war. Die Menge des C1-INH-Proteins wurde durch NOR-Partigen, Behring, bestimmt, wobei Anti-C1-INH-Antikörper vom Schaf und von Kaninchen benutzt wurden. Die Untersuchung wurde genau nach den Anweisungen des Herstellers durchgeführt. Um das Ausmaß der Komplementaktivierung nach dem Wiederdurchströmen des Transplantats zu vergleichen, wurden auch historische Daten von zwei Gruppen von Affen benutzt, die ein unmodifiziertes oder ein h-DAF transgenisches Transplantat, aber keine ergänzende C1-INH-Behandlung erhalten hatten [34]. Die sofortige postoperative Immunsuppression bestand bei diesen Tieren ebenfalls aus CyP, CyA und niedrigen Dosierungen von Steroiden, jedoch wurde diesen Tieren kein Mycophenolat-Mofetil gegeben.

### Ergebnisse

Zur pharmakokinetischen Analyse wurde i.v. eine Einzeldosis von C1-INH zwei gesunden Cynomolgusaffen gegeben (Versuchstiere Nr. 1 und Nr. 2). Basierend auf Erfahrungen mit der C1-INH-Verabreichung in Menschen [35, 36] wurde eine Dosierung von 150 I.U/kg. gewählt. Die pharmakokinetischen Daten zeigten eine Konzentration von mehr als 200% des Normalwertes. Die berechnete Halbwertszeit betrug 72 bzw. 90 Stunden. Dementsprechend wurde eine Dosierung von 500 I.U. C1-INH, die 1 Stunde nach dem Wiederdurchströmen gegeben wurde und 80 I.U./kg täglich danach bei den folgenden Nieren-Xenotransplantationen von Affen verwendet.

In drei Affen wurde eine lebensunterstützende Schweinenieren-Xenotransplantation durchgeführt. Die nachoperative Immunosuppression umfasste CyA, MMF und Steroide. In den Tieren Nr. 3 und Nr. 4 wurde zusätzlich eine CyP-Induktion verabreicht in Übereinstimmung mit einem Protokoll, das früher in einem anderen Laboratorium bereits festgelegt worden war [37]. Dem Tier Nr. 5 wurde keine CyP-Induktion gegeben. Allen drei Affen wurden ergänzend C1-INH (250 I.U.) täglich nach der Transplantation verabreicht. Nach der Transplantation lagen die systemischen C1-Werte in dem Bereich von 170 bis 210% der Normalwerte. Es gab eine bemerkenswert geringe Abweichung in den Gesamt-C1-INH-Spiegeln zwischen den einzelnen Individuen, insbesondere während der ersten 5 Tage nach der Operation.

Keines der drei Fremdtansplantate wurde hyperakut abgestoßen. Alle drei Schweinenieren zeigten anfänglich eine Harnproduktion; eine stabile Nierenfunktion wurde im nachoperativen Verlauf erzeugt, wobei geringste Kreatininmengen von 31, 71 bzw. 64 µmol/l gefunden wurden. In allen drei Tieren zeigte die Analyse der systemischen Antiporcin-Antibodyspiegel durch FACS keine signifikanten Zunahmen des Titers während der Beobachtungszeit.

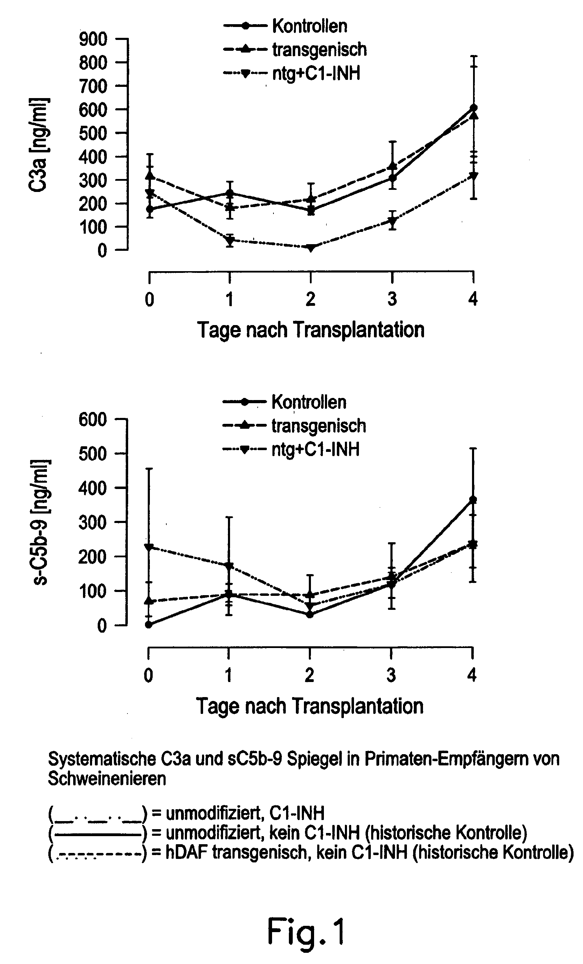

Die Plasmaspiegel von C3a und sC5b-9 wurden in den drei Affen durch ELISA gemessen. Diese Daten wurden mit einem Satz von historischen Daten verglichen, die in Affen nach einer unmodifizierten (n = 3) oder hDAF-transgenen (n = 10) Nierentransplantation ohne kontinuierliche Verabreichung einer C1-INH-Therapie gegeben wurden (Fig. 1). Während die sC5b-9-Spiegel in allen Gruppen vergleichbar waren, tendierten die C3a-Spiegel in der C1-INH-Gruppe, verglichen mit den beiden anderen Gruppen, zu niedrigeren Werten.
Alle drei Affen wurden am 6., 13. und 15. Tag nach der Transplantation wegen einer bakteriellen Sepsis bei stabilen Nierenfunktionen (Urinbildung, Serumkreatininspiegel) verloren. Die Sepsis reagierte nicht auf antimikrobielle Behandlung. In allen drei Affen wurden positive Blutkulturen für E.coli/E.faecalis (Tier Nr. 3) Streptococcus ssp. (Tier Nr. 4) und Streptococcus aureus (Tier Nr. 5) gefunden. Während in allen drei Affen stabile Nierenfunktionen nach der Transplantation gefunden wurden, zeigten sich in den Tieren Nr. 3 und Nr. 4 jedoch ein leichter Anstieg des Kreatininspiegels. In allen Affen wurden Beeinträchtigungen des Koagulationssystems mit einer Erhöhung der partiellen Thromboplastinzeit, vermehrten Fibrinogenabbauprodukten und verringerten AT-III Konzentrationen gefunden. In einem Tier (Tier Nr. 5) waren klinische Zeichen der DIC in allen organischen Systemen bei einer Autopsie sichtbar, während in den anderen zwei Tieren - trotz erheblicher Erhöhung der Fibrinogenabbauprodukte - keine Zeichen von klinisch manifesten Koagulationsstörungen beobachtet wurden.

### Literaturverzeichnis:

- 1.: Schapira M. et al., 1985, Complement 2: 111
- 2.: Davis A.E., 1988, Ann Rev Immunol 6: 595
- 3.: Sim R.B. et al., 1979, FEBS Lett 97: 111
- 4.: De Agostini A. et al., 1984, J Clin Invest 73: 1542
- 5.: Fixley R.A. et al., 1985, J Biol Chem 260: 1723
- 6.: Schapira M. et al., 1982, J Clin Invest 69: 462
- 7.: Van der Graaf F. et al., 1983, J Clin Invest 71: 149
- 8.: Harpel P.C. et al., 1975, J Clin Invest 55: 593
- 9.: Meijers J.C.M. et al., 1988, Biochemistry 27: 959
- 10.: Wuillemin W.A. et al., 1995, Blood 85: 1517
- 11.: Harpel P.C. et al., 1975, J Clin Invest 55: 149
- 12.: Booth N.A. et al., 1975, Blood 69:1600
- 13.: Bock S.C. et al., 1986, Biochemistry 25: 4292
- 14.: Carter P.E. et al., 1988, Eur J Biochem 173: 163
- 15.: Eldering E. et al., 1988, J Bio Chem 263: 11776
- 16.: Eldering E. et al., 1993, J Biol Chem 267: 7013
- 17.: Eldering E. et al., 1993, J Clin Invest 91: 1035
- 18.: Davis A.E. et al., 1992, Nature Genetics 1: 354
- 19.: Eldering E. et al., 1995, J Biol Chem 270: 2579
- 20.: Verpy et al., 1995, J Clin Invest 95: 350
- 21.: Travis J. et al., 1983, Ann Rev Biochem 52: 655
- 22.: Carrel R.W. et al., 1985, Trends Bioch Sci 10: 20
- 23.: Schapira M. et al., 1985, Complement 2: 111
- 24.: Nuijens JH et al., 1989, J Clin Invest 84: 443
- 25.: Kalter ES et al., 1985, J. Infect, Dis 151: 1019
- 26.: Travis J. et al., 1983, Ann Rev Biochem 52: 655
- 27.: Zaidi A, Schmoeckel M, Bhatti F, et al. Life-supporting pig-to-primate renal xenotransplantation using genetically modified donors. Transplantation 1998: 65: 1584.
- 28.: Platt JL, Fischei RJ, Matas AJ, et al. Immunopathology of hyperacute xenograft rejection in a swine-to-primate model. Transplantation 1991: 52: 214.
- 29.: Dalmasso, A.P., Vercellotti, G.M., Fischel, R.J. et al., Mechanism of complement activation in the hyperacute rejection of procine organs transplanted into primate recipients. AM J. Pathol 1991: 140: 1157.
- 30.: Davis EA, Pruitt SK, Greene PS, et al. Inhibition of complement, evoked antibody, and cellular response prevents rejection of pig-to-primate cardiac xenografts. Transplantation 1996: 62: 1018.
- 31.: Waterworth PD, Cozzi E, Tolan MJ, et al. Pig-to-primate cardiac xenotransplantation and cyclophosphamide therapy. Transplant Proc 1997: 29: 899
- 32.: Valdivia L.A., Murase N., Rao A.S., et al.: Use of Tacrolimus (FK 506) and Antimetabolites as Immunosuppressants for Xenotransplantation across closely related rodent species. In: Cooper, Kemp, Platt, White (eds.): Xenotransplantation. The Transplantation of Organs and Tissues between Species. Springer, Bedin, 1996;328.
- 33.: Loss M, Kunz R, Przemeck M, et al. Influence of cold ischemia time, pretransplant anti-porcine antibodies and donor/recipient size matching on hyperacute graft rejection following discordant porcine to cynomolgus kidney transplantation. Transplantation 2000;69:1155.
- 34.: Loss M, Vangerow B, Schmidtko J, et al.: Acute vascular rejection is associated with systemic complement activation in a pig-to-primate kidney xenograft model. Xenotransplantation 2000;7:186.
- 35.: Radke A, Mottaghy K, Goldmann C, Khorram-Sefat R, Kovacs B, Janssen A, Klosterhalfen B, Hafemann B, Pallua N, Kirschfink M : Cl inhibitor prevents capillary leakage after thermal trauma. Crit Care Med 2000 Sep;28(9):3224-32
- 36.: G.Marx, B.Nashan, M.Cobas Meyer, B.Van-gerow, H.J.Schlitt, S.Ziesing, M.Leuwer, S.Piepenbrock, H.Rueckoldt: Septic shock after liver Transplantation for Caroli's disease: clinical improvement after treatment with Cl-esterase inhibitor. Intensive Care Med 1999,25:1017
- 37.: Soin B, Ostlie D, Cozzi E, Smith KG, Bradley JR, Vial C, Masroor S, Lancaster R, White DJ, Friend PJ: Growth of porcine kidneys in their native and xenograft environment. Xenotransplantation 2000 May;7(2):96-1 00
- 38.: Fiane AE, Videm V, Johansen HT, et al. CI-INHibitor attenuates hyperacute rejection and inhibits complement, leukocyte and platelet activation in an ex vivo pig-to-human perfusion model. Immunopharmacology 1999: 42: 231.
- 39.: Europäische Patentanmeldung 0 586 909
- 40.: Internationale Patentanmeldung WO 91/06650 (Cetus Corporation)
- 41.: Internationale Patentanmeldung WO 97/16064
- 42.: Internationale Patentanmeldung WO 92/22320 (Genentech Inc.)

## Patentansprüche

1. Verwendung von humanem C1-Esterase-Inhibitor (C1-INH) zur Herstellung eines Arzneimittels zur Verhinderung oder Verzögerung der hyperakuten und /oder akuten Abstoßung von Xenotransplantaten in Säugetieren
**dadurch gekennzeichnet, dass** das Arzneimittel dem empfangenden Organismus bereits vor der Transplantation verabreicht wird und er nach erfolgter Transplantation tägliche Dosierungen der C1-INH-Zubereitung in einer Menge erhält, die zur Verhinderung einer Abstoßung des Transplantats ausreicht.

2. Verwendung nach den Anspruch 1,
**dadurch gekennzeichnet, dass** die vor der Transplantation verabreichte Menge an C1-INH nicht mehr als 150 I.U./kg Körpergewicht beträgt.

3. Verwendung nach den Ansprüchen 1 bis 2,
**dadurch gekennzeichnet, dass** die nach erfolgter Transplantation verabreichte Menge an C1-INH nicht mehr als 80 I.U./kg Körpergewicht beträgt.

4. Verwendung nach den Ansprüchen 1 bis 3,
**dadurch gekennzeichnet, dass** das Xenotransplantat vom Schwein stammt und der Empfänger ein Primat ist.

5. Verwendung nach Anspruch 4,
**dadurch gekennzeichnet, dass** der Empfänger ein Mensch ist.

## Claims

1. Use of human C1 esterase inhibitor (C1-INH) for producing a medicament for preventing or delaying hyperacute and/or acute rejection of xenotransplants in mammals, **characterized in that** the medicament is administered to the recipient organism before the transplantation and, after the transplantation has taken place, the organism receives daily doses of the C1-INH preparation in an amount sufficient to prevent rejection of the transplant.

2. Use according to Claim 1, **characterized in that** the amount of C1-INH administered before the transplantation is not more than 150 I.U./kg of body weight.

3. Use according to Claims 1 to 2, **characterized in that** the amount of C1-INH administered after the transplantation has taken place is not more than 80 I.U./kg of body weight.

4. Use according to Claims 1 to 3, **characterized in that** the xenotransplant is of porcine origin and the recipient is a primate.

5. Use according to Claim 4, **characterized in that** the recipient is a human.

## Revendications

1. Utilisation de l'inhibiteur de la C1-estérase (C1-INH) humaine pour préparer un médicament destiné à empêcher ou retarder le rejet hyper-aigu et/ou aigu de xénogreffes chez des mammifères, **caractérisée en ce que** le médicament est administré à l'organisme receveur déjà avant la transplantation, ce dernier recevant, après la réussite de la transplantation, des posologies journalières de la préparation de C1-INH en une quantité qui est suffisante pour empêcher un rejet de la greffe.

2. Utilisation selon la revendication 1, **caractérisée en ce que** la quantité de C1-INH administrée avant la transplantation n'est pas supérieure à 150 U.I./kg de poids corporel.

3. Utilisation selon les revendications 1 à 2, **caractérisée en ce que** la quantité de C1-INH administrée après que la transplantation a été effectuée n'est pas supérieure à 80 U.I./kg de poids corporel.

4. Utilisation selon les revendications 1 à 3, **caractérisée en ce que** la xénogreffe provient du porc, et le receveur est un primate.

5. Utilisation selon la revendication 4, **caractérisée en ce que** le receveur est un humain.
